(19) Europäisches Patentamt
European Patent Office
Office européen des brevets

(11) Publication number: 0 131 415
A2

# (12) EUROPEAN PATENT APPLICATION

(21) Application number: 84304431.4

(22) Date of filing: 28.06.84

(51) Int. Cl.[4]: C 12 P 21/00
C 12 N 15/00, C 07 K 15/04
B 01 D 15/08, B 01 J 20/22

(30) Priority: 28.06.83 JP 115197/83

(43) Date of publication of application: 16.01.85 Bulletin 85/3

(84) Designated Contracting States: DE FR GB IT

(71) Applicant: KYOWA HAKKO KOGYO KABUSHIKI KAISHA
6-1, Ohte-machi 1-chome
Chiyoda-ku Tokyo 100(JP)

(72) Inventor: Yoshida, Hajime
9-18, Isobe
Sagamihara-shi Kanagawa-ken(JP)

(72) Inventor: Hanai, Nobuo
3-7-10, Yurigaoka
Kawasaki-shi Kanagawa-ken(JP)

(74) Representative: Watkins, Arnold Jack et al,
European Patent Attorney Frank B. Dehn & Co. Imperial House 15-19 Kingsway
London WC2B 6UZ(GB)

## (54) Monoclonal antibodies, processes for their preparation and methods for their use.

(57) Monoclonal antibodies specific for bovine serum albumin are prepared by immunizing a mammal with bovine serum albumin to produce cells capable of producing antibodies in the body of said mammal; fusing said cells with lined myeloma cells originating from a mammal other than the immunized mammal, selecting the resultant fused cells for the production of antibodies specific for bovine serum albumin, subjecting the selected fused cells to cloning to produce monoclones, selecting the resultant monoclones for reactivity with at least one member selected from bovine serum albumin and other related albumins, culturing the selected monoclones and recovering the desired monoclonal antibody from the culture thereby obtained.

Such monoclonal antibodies specific for bovine serum albumin are used in processes for isolating bovine serum albumin from a bovine serum albumin-containing material.

EP 0 131 415 A2

"Monoclonal antibodies, processes for their preparation and methods for their use"

The present invention relates to monoclonal antibodies specific for bovine serum albumin, processes for their preparation and methods for their use.

Bovine serum albumin is a type of protein present in bovine serum. Its molecular weight is 66,000. Usually, serum of adult cattle, calves or foetal cattle is added to various culture media. Even when such a serum is removed from the culture medium, the presence of bovine serum albumin is often retained as a source of protein. Thus, where any useful substance is produced by culturing animal tissues and is then purified or isolated from the tissue culture, it is sometimes necessary to remove bovine serum albumin from the product by a known method. However, in many cases, the bovine serum albumin cannot necessarily be easily removed. It is known that antisera (polyclonal antibodies) having a degree of specificity for bovine serum albumin may be induced by immunizing animals of different species such as mice, and then recovered from the animal and used for the removal of bovine serum albumin. However, the antisera obtained are not uniform and there is moreover the further difficulty of poor specificity in that the antisera may be reactive with other serum albumins originating from other animals such as human serum albumin.

The present invention is based on the discovery of monoclonal antibodies specific for bovine serum albumin and processes for their preparation and on the discovery that such monoclonal antibodies may be used for isolating bovine serum albumin from a bovine serum albumin-containing material.

Thus according to one feature of the present invention there are provided monoclonal antibodies specific for bovine serum albumin. The monoclonal antibodies are preferably of the IgG isotype, especially the sub-class $IgG_1$.

The monoclonal antibodies of the present invention are advantageously in substantially pure form and may if desired be in the form of an antiserum.

According to a further feature of the present invention there are provided monoclones for the preparation of monoclonal antibodies of the present invention. The monoclones are preferably prepared according to the process described hereinafter for the preparation of the monoclonal antibodies of the present invention or by such steps thereof as are necessary. Selection of the monoclones may for example be effected on the basis of their reactivity with bovine serum albumin and their lack of reactivity with other related albumins.

According to a still further feature of the present invention there is provided a process for preparing a monoclonal antibody of the present invention which comprises immunizing a mammal with bovine serum albumin to produce cells capable of producing antibodies in the body of said mammal, fusing said cells with lined myeloma cells originating from a mammal other than the immunized mammal, selecting the resultant fused cells for the production of antibodies specific for bovine serum albumin, subjecting the selected fused cells to cloning to produce monoclones, selecting the resultant monoclones for reactivity with at least one member selected from bovine serum albumin and other related albumins, culturing the selected monoclones and recovering the desired monoclonal antibody from the culture thereby obtained.

The monoclonal antibodies of the present invention may be prepared in the following manner.

(1) Preparation of immunized animal cells:

Mammals such as rats, mice, guinea pigs, rabbits, goats, horses, cattle and the like may be immunized e.g. in conventional manner with bovine serum albumin. Those cells produced which are capable of producing the corresponding antibodies are collected from the immunized mammal.

For example, bovine serum albumin (20-400 μg per animal) may be injected into the vein or abdominal cavity of a mouse (8-10 weeks old) optionally together with an appropriate adjuvant such as complete Freund's adjuvant, aluminium hydroxide gel, pertussis vaccine and the like, the bovine serum albumin and adjuvant preferably being present in equal amounts. Advantageously subsequent immunizations may be effected for example 2-10 times with an interval of e.g. 1-2 weeks. For example 3-7 days after each immunization, blood may be collected, e.g. from the retinal artery plexus and used to investigate the titre of anti-bovine serum albumin antibody e.g. by means of enzymoimmunoassay techniques such as the solid phase sandwich method [see the method described in "Koso Men-eki Sokutei-ho", published by Igaku Shoin, Japan (1978)] in the following manner.

1% bovine serum albumin (BSA) dissolved in a phosphate buffer solution (PBS) [bovine serum albumin 10g; disodium phosphate 1.83 g; monopotassium phosphate 0.21g; sodium chloride 7.65g and distilled water one litre; pH=7.2] is poured into each well of a 96-well EIA plate (commercial product of Flow Laboratories, U.S.A.) in an amount of 100-200 μl per well and allowed to stand at 4°C overnight. After removal of the BSA/PBS solution, each well is washed with deionized water. Then, as a primary antibody, human serum albumin multiply diluted with PBS [containing mouse serum, supernatant of the hybridoma cells and crude monoclonal antibodies [100 µl per well] is allowed to stand at 4°C overnight. The EIA plate is washed once with deionized water and six times with 2M-NaCl. After this, as a secondary antibody, a combined product of anti-mouse immunoglobulin $F(ab)_2$ with urease [commercial product of Commonwealth Serum Laboratories, Australia (CSL); diluted to X100] is poured into each well in an amount of 100 µl per well and allowed to stand at room temperature for 2 hours. The plate is washed 3 times with deionized water, and then an urease substrate solution (commercial product of CSL, Australia; is poured into each well in an amount of 100 µl per well and allowed to stand at room temperature for 10-30 minutes. After this, the reaction is discontinued for example by adding 1% methylthiolate (20 µl per well) to the reaction mixture.

The antibody titre is conveniently determined by colorimetric determination at 600 nm.

Where the antibody titre of the serum derived from any mouse reaches more than $10^3$ times the corresponding titre of mouse normal serum against bovine serum albumin, such a mouse may be regarded as a useful source of immunized animal cells and may be term "qualified".

In order to prepare the hybridoma cells, bovine serum albumin (20-400 µg per animal) is preferably administered to the immunized animal 3-4 days before hybridization. Then, for example, the spleen cells may be collected from the animal in conventional manner and used to prepare the desired hybridoma cells.

(2) Preparation of myeloma cells:

Suitable lined cells of mouse origin may conveniently be used as the myeloma cells, for example myeloma cells of 8-azaguanine-resistant mice (of BALB/c strain), such as P3-X63-Ag8-Ul (P3-Ul) [Current Topics in Microbiology and Immunology, 81. 1-7 (1978)], P3-NSI/1-Ag4.1 (NS-1) [European J. Immunology, 6, 511-519 (1976)], SP2/0-Ag14 (SP-2) [Nature, 276, 269-270 (1978)], P3-X63-Ag8.653 (653) [J. Immunology, 123, 1548-1550 (1979)], P3-X63-Ag8 (X63) [Nature, 256, 495-497 (1975)] and the like. These cell lines may be subcultured, for example, by using a known 8-azaguanine medium. 3-4 Days before hybridization, the myeloma cells are preferably subcultured, for example by using a normal medium so as to ensure the number of cells is more than 2 X $10^7$ on the date of hybridization.

(3) Fusion:

For example, the spleen cells thus obtained and the myeloma cells obtained by the above-mentioned step (2) are mixed together, the ratio of the number of spleen cells to the number of myeloma cells being preferably 5-10:1. The cells are fused in conventional manner, for example by using a mixed solution of polyethylene glycol 1000 (PEG 1000) (1-4 g), MEM (1-4 ml) and dimethylsulfoxide (0.5-1 ml) conveniently in an amount of 0.1-1 ml per $10^8$ spleen cells.

The cell suspension is poured into each well of a culture plate for incubation at 35-40°C for 10-30 hours in an incubator containing 3-7% $CO_2$. To each well of the plate is added a HAT medium for further culturing for 10-14 days in conventional manner. During the culturing the HAT medium is replaced for example 3 times with a new HAT medium. After this, the medium is replaced by HT medium in conventional manner for further culturing.

After culturing for example for 3-4 days by using HT medium, part of the supernatant is collected to determine the titre of anti-bovine serum albumin antibody e.g. by the above-mentioned enzymoimmunoassay method. If desired, radioimmunoassay may be used solely or in combination therewith. Similarly, the reactivities of the fused cells with human serum albumin, egg white albumin and the like are also investigated to select those which are specifically reactive with bovine serum albumin. The fused cells which are strongly reactive with bovine serum albumin and unreactive with human serum albumin and egg white albumin are selected. They are subjected to cloning e.g. twice by the limiting dilution method to obtain hybridoma cells which are stable and capable of producing anti-bovine serum albumin antibodies.

(4) Preparation of monoclonal antibodies:

The hybridoma cells, recognized as those capable of producing anti-bovine serum albumin antibodies, are, for example, administered in an amount of 2-4 X $10^6$ cells/animal to a pristan (2,6,10,14-tetramethylpentadecane)-treated BALB/c mouse (female; 8-10 weeks old) for example by abdominal injection. 10-21 days after this, ascites tumour is induced by the hybridoma cells. The ascites collected from the mouse is preferably centrifuged to remove solid impurities, and the salting-out of the supernatant is conveniently effected twice e.g. with 50% and 40% ammonium sulfate respectively, followed by for example dialysis for 1-2 days against a PBS solution (pH=7.2). The resultant residual fraction may be passed through a column packed, for example, with DEAE-cellulose, protein A Sepharose 4B and the like to collect IgG fractions which may then be used as purified monoclonal antibodies.

The binding activity and inhibiting activity of the monoclonal antibody are assayed for example by the above-mentioned enzymoimmunoassay method in order to investigate the specificity of the monoclonal antibody.

The isotype of the monoclonal antibody may be determined by Ouchterlony's method (double diffusion test) [see "Men-eki-gaku jikken Nyumon, Seibutus-kagaku Jikken-ho, 15, page 74 (1981)" published by Gakkai Shuppan Centre, Japan].

Quantitative determination of protein may be effected according for example to the method of Folin and the optical density at 280 nm [1.4 $(OD_{280}) \fallingdotseq$ immunoglobulin 1 mg/ml].

In this manner, we have found that the monoclonal antibody which we have designated KM-10 and which is obtained from hybridoma cells which we have designated KM-10 hybridoma cells is of the $IgG_1$ isotype, and its specificity is shown in Example 1 described hereinafter.

According to a further feature of the present invention there is provided a process for isolating bovine serum albumin from a bovine serum albumin-containing material, which process comprises adsorbing bovine serum albumin using a monoclonal antibody of the present invention.

The process thus provides for example a method for removing bovine serum albumin from a culture medium, for example containing a desired substance e.g. a proteinaceous substance such as interferon $\alpha$ or a method of purifying a desired substance e.g. a proteinaceous substance such as interferon $\alpha$ which for example has been prepared by culturing and has been recovered therefrom, but which is contaminated with bovine serum albumin. The process is conveniently effected by affinity chromatography known *per se*.

The monoclonal antibodies of the present invention are conveniently immobilized by the use of an appropriate carrier e.g. by reaction with a cyanogen bromide-activated exchange resin e.g. CNBr-Sepharose 4B.

In a further embodiment of the present invention there is provided a composition, e.g. in the form of an affinity column, for adsorbing bovine serum albumin comprising a monoclonal antibody of the present invention immobilized on a carrier therefor. Such a composition according to the invention may be used for any convenient purpose as described above such as the purification of a desired substance obtained by culturing animal tissue e.g. interferon-α.

Thus by using the monoclonal antibodies of the present invention, bovine serum albumin may be removed from proteinaceous substances containing bovine serum albumin, for example, in the following manner:-

A monoclonal antibody of the present invention e.g. KM-10 (10mg) may for example be dissolved in a PBS solution (e.g. 1-10 ml) and then for example subjected to reaction with an appropriate carrier such as for example CNBr-activated Sepharose 4B (1 ml; commercial product of Pharmacia Fine Chemicals AB., Sweden) to obtain an immobilized monoclonal antibody. The resultant immobilized monoclonal antibody may then be packed into a column and a solution of bovine serum albumin (not more than 5 mg) may then be passed through the column. In this manner, it is possible to adsorb 99-100% of bovine serum albumin on the column. Where a sample containing proteins other than bovine serum albumin is passed through the column, such other proteins are not adsorbed by the column. Where the ratio of monoclonal antibody to bovine serum albumin is over the range 2:1 by weight, a single passage through the column may result in almost complete removal of the bovine serum albumin contained in the sample solution. It is also possible to remove the bovine serum albumin contained in the column almost completely, for example, by eluting with a 0.01-0.1M glycine-HCl buffer solution and the like so that it is possible to reuse the antibody column of the present invention, for example, 5-10 times. If desired the bovine serum albumin may be recovered from the eluant.

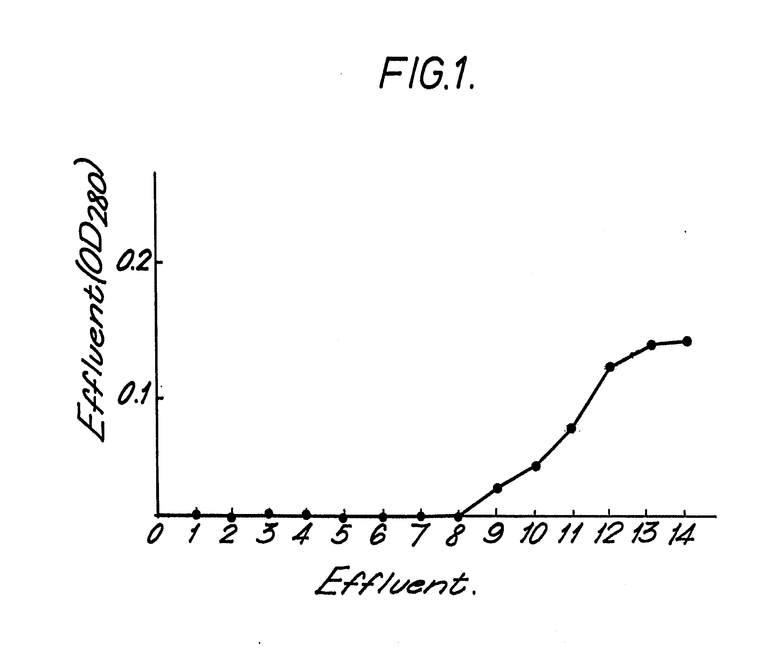

With regard to the drawing, Figure 1 shows that a column containing 10g of immobilized monoclonal antibody of the present invention is capable of completely adsorbing bovine serum albumin up to an amount of 4 mg, after which the column gradually becomes saturated and further bovine serum albumin ceases to be effectively adsorbed. The drawing plots volume of effluent against optical density (absorbance) at 280 nm and reflects the result of Example 2 described hereinafter.

Example 1

(1) Preparation of the spleen cells of immunized mice:

5 Female mice (BALB/c strain; 8 weeks old; purchased from Shizuoka-ken Jikken Dobutsu Nogyo Kyodo Kumiai, Japan) were immunized by abdominal injection of bovine serum albumin (100 μg/animal; commercial product of Fraction V. Sigma, U.S.A.) in association with as adjuvants aluminium hydroxide gel (2 mg/animal) and inactivated pertussis vaccine (1 X $10^9$ cells/animal; commercial product of Chiba-ken Kessei Kenkyusho, Japan). Subsequent immunizations were effected after an interval of one week by abdominal injection of bovine serum albumin (100 μg/animal on each occasion). After the tertiary immunization, 5-7 days after each immunization, blood was collected from the retinal artery plexus of the eye to investigate the titre of the anti-bovine serum albumin antibody by the above-mentioned solid phase method for enzymoimmunoassay.

One week after the tertiary immunization, all mice showed positive antibody titres. However, additional immunizations were carried out twice, and thus all animals were immunized 5 times in total. 3 Days after the final immunization, the spleen was collected from each mouse in conventional manner and used for fusion of the cells.

(2) Preparation of mouse myeloma cells:

P3-Ul (myeloma cells of 8-azaguanine-resistant mice) were cultured by using a normal medium [RPMI-1640 containing glutamine (1.5 mM), 2-mercaptoethanol (5 X $10^{-5}$M), gentamicin (10 μg/ml) and foetal calf serum (0.1 ml/ml)] for 4 days to obtain more than 2 X $10^7$ cells.

(3) Preparationof hybridoma cells:

The spleen cells of immunized mice (1 X $10^8$) were

washed with MEM medium (commercial product of Nissui Seiyaku K.K., Japan) and mixed with 1 X $10^7$ P3-U1 myeloma cells originating from 8-azaguanine-resistant mice. The mixture was centrifuged (1200 r.p.m./5 minutes) to give a precipitate of the mixed cells. The mixed cells were loosened, and a solution (0.5 ml) containing a mixture of polyethylene glycol-1000 (PEG-1000; 2 g), MEM medium (2 ml) and dimethylsulfoxide (0.7 ml) was then added thereto. One minute after this, MEM medium (1 ml) was added to the cell suspension. Subsequently, MEM medium was added to the cell suspension five times (1 ml each time) with an interval of one minute. After this, the total amount of MEM medium in the suspension was made up to 50 ml with further addition of the same medium. The suspension was centrifuged (900 r.p.m.) to remove the supernatant. The resultant mass of cells was loosened, a normal medium (100 ml) added thereto and the cells loosened by using a messpipet.

The cell suspension was poured into the wells of a 24-well culture plate (commercial product of Flow Laboratory, U.S.A.) in an amount of one ml per well, followed by incubating at 37°C for 24 hours by using a $CO_2$ incubator. After adding to the plate a HAT medium [prepared by adding hypoxanthine ($10^{-4}$M), thymidine (1.5 X $10^{-5}$M) and aminopterin (4 X $10^{-7}$M), the culturing was effected for 24 hours. After removal of the supernatant (1 ml), a new HAT medium (1 ml) was added to each medium for further culturing at 37°C for 10-14 days.

The presence of the fused cells grown in the form of colonies was found in certain wells, from which, on each occasion, the supernatant (1 ml) was removed and replaced by new HT medium (1 ml; prepared by removing aminopterin from HAT medium as above-mentioned) for culturing at 37°C. Such replacement by HT medium and further culturing were continued for the next 4 days. Then part of the supernatant was collected from each of the qualified cultures for assaying the titre of the anti-bovine serum albumin antibodies by the solid phase enzymoimmunoassay method as hereinbefore described.

The fused cells exhibiting sufficient antibody titres were collected from the wells and subjected to cloning twice by the limiting dilution method to obtain clones having stability and qualified antibody titres. The resultant monoclones were recognized as the hybridoma cells capable of producing anti-bovine serum albumin antibodies. Among 10 clones obtained, the one having the highest activity was selected and used to prepare monoclonal antibodies, this clone being designated as KM-10.

(4) Partial purification of monoclonal antibodies:

Pristan (2,6,10,14-tetramethyl-pentadecane) (0.5 ml/animal) was abdominally administered to BALB/c female mice (8 weeks old) which were bred for 2 weeks. Then, the above-mentioned hybridoma cells ($4 \times 10^6$ cells/mouse on each occasion) were administered to the mice by abdominal injection. 10-21 Days after this, an ascites tumour was induced by the hybridoma cells. 10-21 Days after this, ascites (4-10 ml/mouse) was collected from the mice, from which solid materials were removed by centrifugation. The salting-out of the supernatant was effected twice using 50 and 40% ammonium sulfate solution respectively, followed by dialysis against a PBS solution (pH=7.2) for 2 days to obtain partially purified monoclonal antibodies.

The residual solution (10.2 ml; 31 g/ml) was chromatographed by using a column (1.1 X 20 cm) packed with DEAE-cellulose (76 ml; commercial product of Pharmacia Fine Chemicals AB., Sweden). The effluent was divided into fractions (each 1 ml). The fractions containing IgG (Nos. 4-5) were collected and combined to obtain a purified monoclonal antibody, the presence of a single antibody being confirmed by electrophoresis.

(5) Antigenic specificity of the purified monoclonal antibody:

The antigenic specificity of the purified monoclonal antibody was assayed by the solid phase method for assaying the immunological enzyme activity. As antigens, bovine serum albumin (BSA) (commercial product of Sigma U.S.A.), human serum albumin (HSA) (commercial product of Midori Juji K.K., Japan), casein (CA) and egg white albumin (OVA) (commercial product of Sigma, U.S.A.) and foetal calf serum (FCS) (commercial product of CSL, Australia) were used to obtain the results shown in Table 1.

TABLE 1

| Antibody | Diluted | Binding activity ($OD_{600}$) | | | | |
|---|---|---|---|---|---|---|
| | | BSA | HSA | CA | OVA | FCS |
| Normal mouse serum | $10^{-2}$ | 0.010 | 0.003 | 0.005 | 0.050 | 0.007 |
| | $10^{-3}$ | 0.003 | 0.005 | 0.004 | 0.033 | 0.003 |
| | $10^{-4}$ | 0.002 | 0.002 | 0.005 | 0.005 | 0.003 |
| BSA-immunized mouse serum | $10^{-2}$ | 0.870 | 0.435 | 0.231 | 0.535 | 0.785 |
| | $10^{-3}$ | 0.845 | 0.215 | 0.090 | 0.480 | 0.670 |
| | $10^{-4}$ | 0.820 | 0.085 | 0.045 | 0.110 | 0.400 |
| KM-10 purified monoclonal antibody | $10^{-2}$ | 0.830 | 0.190 | 0.110 | 0.307 | 0.690 |
| | $10^{-3}$ | 0.834 | 0.150 | 0.040 | 0.245 | 0.405 |
| | $10^{-4}$ | 0.845 | 0.045 | 0.000 | 0.092 | 0.400 |
| | $10^{-5}$ | 0.280 | 0.002 | 0.000 | 0.050 | 0.395 |
| | $10^{-6}$ | 0.040 | 0.000 | 0.000 | 0.040 | 0.050 |

The inhibiting activity of bovine serum albumin, human serum albumin, casein, egg white albumin or foetal calf serum against the binding of the monoclonal antibody KM-10 of the present invention with bovine serum albumin was assayed in the following manner. An antibody was mixed with an inhibitor for reaction at 37°C for 30 minutes. The reaction product is used as a primary antigen for the enzyme immunization method. The results are shown in Table 2.

TABLE 2

| Inhibitor | Concentration (%) | Inhibition rate (%) |
|---|---|---|
| BSA | 0.01 | 90.9 |
| | 0.001 | 80.7 |
| HSA | 0.01 | 0.0 |
| | 0.001 | 0.0 |
| CA | 0.01 | 0.0 |
| | 0.001 | 0.0 |
| OVA | 0.01 | 0.0 |
| | 0.001 | 0.0 |
| FCS | 0.01 | 85.1 |
| | 0.001 | 9.6 |
| None | | 0.0 |

This table indicates that the monoclonal antibody of the present invention exhibits very high specificity for bovine serum albumin.

(6) Classification of monoclonal antibody:

Ouchterlony's method was applied to investigate the isotype of KM-10 and it was found that the monoclonal antibody of the present invention is of the IgG class, subclass $lgG_1$.

Example 2

Monoclonal antibody KM-10 specific for bovine serum albumin was prepared by the method of Example 1. 10 mg of KM-10 [in a carbonate buffer solution containing 0.1M $NaHCO_3$ and 0.5M NaCl and having a pH of 8.3] was reacted with CNBr-activated Sepharose 4B (1 ml; commercial product of Pharmacia Fine Chemicals AB., Sweden) at room temperature for 2 hours to prepare an immobilized monoclonal antibody. The resultant immobilized monoclonal antibody was packed in a column (1 ml), and 0.5 mg/ml of bovine serum albumin in a carbonate buffer solution (0.1M $NaHCO_3$ and 0.5M NaCl; pH 8.3) was passed through the column at a velocity of 6.7 ml/hour. The effluent was divided into small fractions (each 1 ml), and the absorbances of each fraction at 280 nm are shown in Fig. 1.

From Fig. 1, it is apparent that a column containing 10mg of the immobilized monoclonal antibody is capable of completely adsorbing up to 4 mg in total of bovine serum albumin (0.5 mg/ml X 8 ml).

Furthermore it is also possible to recover the bovine serum albumin adsorbed onto the column by passing a 0.04M glycine-hydrochloric acid buffer solution (pH=2.3) through the column, the recovery ratio being nearly 100%. The column may thus be reused.

Example 3

A similar KM-10 column to that described in Example 2 was used under similar conditions. A solution of a carbonate buffer (0.1M $NaHCO_3$ and 0.5M NaCl; pH=8.3; 1 ml) containing bovine serum albumin (1 mg), ribonuclease (1 mg), chymotrypsinogen (1 mg) and egg white albumin (1 mg) was passed through the column and the effluent was investigated by electrophoresis. It was found that ribonuclease, chymotrypsinogen and egg white albumin were not adsorbed and these substances were found in the effluent. Bovine serum albumin was not found in the effluent, but was retained in the column. Only bovine serum albumin was recovered from the climun by passing a 0.04M glycine-hydrochloric acid buffer solution (pH=2.3) through the column. Thus, the column is capable of selectively adsorbing only bovine serum albumin from a mixture of proteins.

Claims:

1. Monoclonal antibodies specific for bovine serum albumin.

2. A monoclonal antibody as claimed in claim 1, of the IgG isotype.

3. A monoclonal antibody as claimed in claim 2 of the $IgG_1$ sub-class.

4. A monoclonal antibody as claimed in any one of the preceding claims in the form of an antiserum.

5. A process for preparing a monoclonal antibody as defined in any one of claims 1-3, which comprises immunizing a mammal with bovine serum albumin to produce cells capable of producing antibodies in the body of said mammal, fusing said cells with lined myeloma cells originating from a mammal other than the immunized mammal, selecting the resultant fused cells for the production of antibodies specific for bovine serum albumin, subjecting the selected fused cells to cloning to produce monoclones, selecting the resultant monoclones for reactivity with at least one member selected from bovine serum albumin and other related albumins, culturing the selected monoclones and recovering the desired monoclonal antibody from the culture thereby obtained.

6. A process according to claim 5, in which the mammal is selected from rats, guinea pigs, rabbits, goats, horses and cattle.

7. A process according to claim 5 in which the mammal is a mouse.

8. A process for isolating bovine serum albumin from a bovine serum albumin-containing material, which comprises adsorbing bovine serum albumin using a monoclonal antibody specific for bovine serum albumin.

9. A composition for adsorbing bovine serum albumin which comprises a monoclonal antibody as defined in any of claims 1 to 3 immobilized on a carrier therefor.

10. A composition according to claim 9 in the form of an affinity column.

11. Monoclones for the preparation of monoclonal antibodies as defined in any one of claims 1 to 3.

12. Monoclones for the preparation of monoclonal antibodies as defined in any one of claims 1 to 3 prepared and selected by a process as defined in any one of claims 5 to 7.

FIG.1.

Effluent (OD280)
0.2
0.1
0
1
2
3
4
5
6
7
8
9
10
11
12
13
14
Effluent.